# EUROPEAN PATENT SPECIFICATION

(11) **EP 2 826 427 B1**
(45) Date of publication and mention of the grant of the patent: **25.04.2018**
(21) Application number: 13761434.3
(22) Date of filing: 05.03.2013
(51) Int. Cl.: A61B 17/00, A61M 35/00, A61M 5/14, A61B 17/34, A61M 5/142, A61M 5/00

(54) **APPLICATOR**
APPLIKATOR
APPLICATEUR

(30) Priority: 15.03.2012 JP 2012058267
(43) Date of publication of application: 21.01.2015
(73) Proprietor: Terumo Kabushiki Kaisha, Tokyo 151-0072 (JP)
(72) Inventor: YOKOYAMA Kenji, Ashigarakami-gun Kanagawa 259-0151 (JP)
(74) Representative: Casalonga
(86) International application number: PCT/JP2013/055983
(87) International publication number: WO 2013/137062

(56) References cited:
- EP-A1- 2 163 204
- EP-A1- 2 248 470
- EP-A1- 2 361 568
- WO-A1-2009/107619
- WO-A1-2010/044462
- JP-A- 2011 182 994
- US-A1- 2006 189 944

## Description

### Technical Field

The present invention relates to an applicator for applying an anti-adhesion material, a biological tissue adhesive or the like to an affected area or the like, and particularly to an applicator suitable for use in a laparoscopic operation.

### Background Art

Conventionally, a method is known wherein two or more kinds of liquids are mixed and injected to an affected area or the like to form an anti-adhesion material, a biological tissue adhesive or the like, and an applicator for the method has been developed.

A conventional applicator is configured such that components which solidify when they are mixed, for example, solution containing thrombin and solution containing fibrinogen are fed in a separated state from each other to a location in the proximity of an affected area and are applied while being mixed at the affected area. EP 2 361 568 discloses an applicator according to the preamble of claim 1.

As such an applicator as just described, an applicator is available which includes two syringes individually containing different kinds of liquids and a nozzle which mixes and jets the liquids from the syringes (for example, refer to Patent Document 1).

The applicator of Patent Document 1 includes a nozzle and a sheath. The nozzle includes a nozzle main body of an elongated tubular shape and a nozzle head provided at a distal end of the nozzle main body. At a distal end portion of the nozzle main body, a curved portion having flexibility and curved or bent is formed. The sheath corrects, when the nozzle main body is fitted into the sheath for movement along a longitudinal axial direction thereof and the curved portion is inserted into the sheath, the shape of the curved portion to adjust the direction of the nozzle head with respect to an axial line of the nozzle main body. A gap is formed in the longitudinal axial direction between the sheath and the nozzle. The gap functions as an exhaust path for exhausting, when the abdominal pressure in the abdominal cavity rises, the gas in the abdominal cavity to the outside of the body through the gap.

Further, on the sheath of the applicator of Patent Document 1, two side holes extending through a wall portion are formed at one position on a circumference at the proximal end side with respect to the opening at the distal end. The side holes are disposed in an opposing relationship to each other across the axis of the sheath at positions on the same circumference with regard to the longitudinal axial direction of the sheath. Each of the side holes functions as an intake port for taking in gas in the abdominal cavity therethrough. In the applicator of Patent Document 1, gas is injected into the abdominal cavity upon treatment in which anti-adhesion material, biological tissue adhesive or the like is used. Although the pressure in the abdominal cavity is raised by the gas, the gas in the abdominal cavity is exhausted to the outside of the body through the side holes.

In this manner, in the applicator, a pressure rise in the abdominal cavity upon treatment in which anti-adhesion material, biological tissue adhesive or the like is used is suppressed by the side holes formed at one position of the sheath at the proximal end side with respect to the opening at the distal end.

### Prior Art Document

### Patent Document

Patent Document 1: U.S. Patent Application Publication No. 2010/0331766

### Summary of Invention

### Technical Problem

However, in the applicator of Patent Document 1, there is the possibility that, if a distal end portion of the sheath is dipped in liquid existing in the abdominal cavity, the opening at the distal end of the sheath and the side holes may be closed up. In this case, there is the possibility that a pressure rise in the abdominal cavity upon the treatment described above may not be able to be suppressed.

It is an object of the present invention to provide an applicator which eliminates the problems based on the prior art described above and can suppress an influence on the pressure in the abdominal cavity irrespective of a usage pattern.

### Technical Solution

In order to achieve the object described above, according to the present invention, there is provided an applicator as defined in claim 1. Preferred embodiments are defined in the dependent claims.

### Advantageous Effect

With the applicator of the present invention, a plurality of side holes are formed on a circumference at each of a plurality of positions spaced by an equal distance along the longitudinal axial direction of the sheath. Therefore, a plurality of exhaust routes for gas in the abdominal cavity to the outside of the body are provided. Consequently, gas in the abdominal cavity can be exhausted to the outside of the body irrespective of the usage pattern of the applicator, and a pressure rise in the abdominal cavity can be suppressed. Brief Description of Drawings

[FIG. 1]

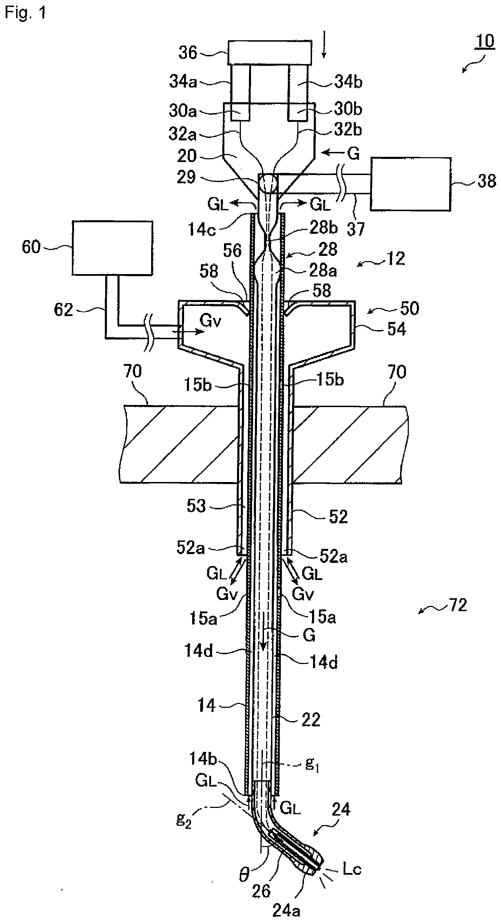

   FIG. 1 is a schematic view depicting an applicator of an embodiment of the present invention.
[FIG. 2]

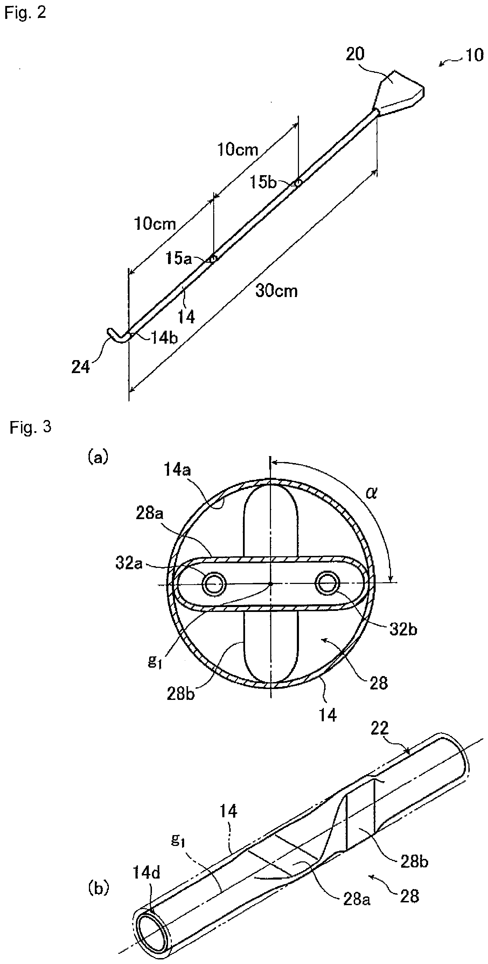

   FIG. 2 is a schematic perspective view depicting the applicator of the embodiment of the present invention.
[FIG. 3]
   FIG. 3(a) is a schematic sectional view depicting a crushed portion of the applicator of the embodiment of the present invention, and FIG. 3(b) is a schematic perspective view depicting the crushed portion of the applicator of the embodiment of the present invention.
[FIG. 4]

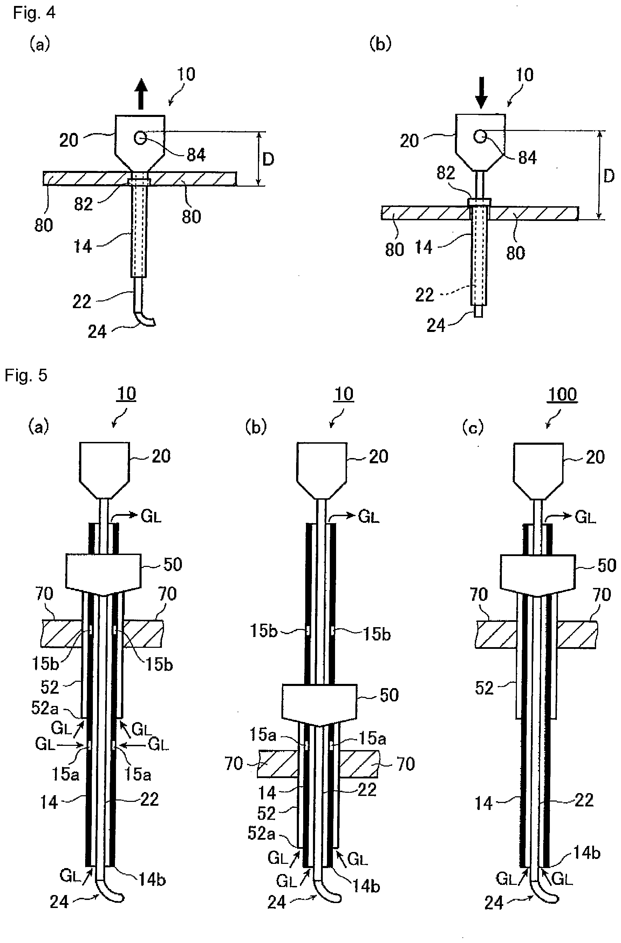

   FIGS. 4(a) and 4(b) are schematic views depicting an apparatus used for a measuring method of sliding resistance.
[FIG. 5]
   FIGS. 5(a) and 5(b) are schematic views illustrating usage patterns of the applicator of the embodiment of the present invention, and FIG. 5(c) is a schematic view of a usage pattern of a conventional applicator.

### Mode for Carrying Out the Invention

In the following, the applicator of the present invention is described in detail based on a preferred embodiment depicted in the accompanying drawings.

FIG. 1 is a schematic view depicting an applicator of an embodiment of the present invention; FIG. 2 is a schematic perspective view depicting the applicator of the embodiment of the present invention; and FIG. 3(a) is a schematic sectional view depicting a crushed portion of the applicator of the embodiment of the present invention and FIG. 3(b) is a schematic perspective view depicting the crushed portion of the applicator of the embodiment of the present invention.

The applicator 10 depicted in FIG. 1 is for being inserted into an abdominal cavity 72 upon laparoscopic operation to apply anti-adhesion material or biological tissue adhesive formed by mixing two kinds of liquids having different compositions from each other to an affected area such as an organ or an abdominal wall 70. The insertion of the applicator 10 into the abdominal cavity 72 is carried out through a trocar 50 indwelled in advance in the abdominal wall 70.

First, the trocar 50 is described.

The configuration of the trocar 50 is not restricted specifically, but various known trocars which are used in laparoscopic operation can be used. For example, it is possible to use the trocar tube disclosed in figure 1 of Japanese Patent Laid-Open No. 2009-226189. Such a trocar 50 as just described is depicted in FIG. 1

As depicted in FIG. 1, the trocar 50 includes a hollow hub 54 communicating with a main body 52 in the form of a pipe. The hub 54 has a diameter greater than that of the main body 52 and is communicated with the main body 52.

An applicator 10 hereinafter described in detail is inserted into the main body 52. When the applicator 10 is inserted in the main body 52, a gap 53 is generated between the main body 52 and a sheath 14 of the applicator 10.

Further, the main body 52 of the trocar 50 may have a distal end opening inclined with respect to an axis of the main body 52. This makes it possible to carry out insertion of the trocar 50 into the abdominal cavity 72 readily.

The hub 54 is connected to a gas supplying unit 60 through a tube 62. The gas supplying unit 60 includes a gas bomb (not depicted) in which sterile gas Gv is filled at a high pressure. From the gas supplying unit 60, the sterile gas Gv is supplied to the abdominal cavity 72 passing the tube 62, the inside of the hub 54 and the inside of the main body 52 in order. An on-off valve (not depicted) for controlling the sterile gas Gv so as to be supplied or stopped is installed in the hub 54, gas supplying unit 60 or tube 62. When sterile gas is to be supplied into the abdominal cavity 72, the valve is placed into an open state. The sterile gas Gv is, for example, the air or nitrogen gas.

The hub 54 has an opening 56 formed at the opposite side thereof to the side at which the main body 52 is provided. A valve 58 is provided in such a manner as to cover the opening 56. The valve 58 is, for example, a duckbill valve. The applicator 10 or the like is inserted into the opening 56 of the trocar 50, and the applicator 10 is inserted into the abdominal cavity 72.

The valve 58 closes up the opening 56 in a state in which the applicator 10 is not inserted in the opening 56, but is opened if the applicator 10 is inserted although the sheath 14 of the applicator 10 and the opening 56 is placed in a sealed state therebetween. Not only in a state in which the applicator 10 is not inserted but also in another state in which the applicator 10 is inserted, flowing out of the sterile gas Gv from the opening 56 can be prevented and the sterile gas Gv is supplied into the abdominal cavity 72 with certainty by the valve 58.

The main body 52 and the hub 54 may be formed integrally with each other or may be formed as separate members which are connected and fixed to each other.

A pressure sensor (not depicted) for measuring the pressure in the abdominal cavity 72 is connected to the trocar 50. A control unit (not depicted) is further provided which causes the sterile gas Gv to be supplied from the gas supplying unit 60 into the abdominal cavity 72 based on the pressure obtained by the pressure sensor. Thus, under the control of the control unit, the sterile gas Gv is supplied from the gas supplying unit 60 into the abdominal cavity 72 until the intraperitoneal pressure (abdominal pressure) in the abdominal cavity 72 is raised by approximately 8 to 12 mmHg from the atmospheric pressure to inflate the abdominal cavity 72. On the other hand, if the pressure in the abdominal cavity 72 drops due to leakage of the gas G_{L} in the abdominal cavity 72, then the sterile gas Gv is supplied from the gas supplying unit 60 into the abdominal cavity 72 under the control of the control unit so that the intraperitoneal pressure is kept at the pressure higher by approximately 8 to 12 mmHg than the atmospheric pressure. The abdominal cavity 72 is controlled to a magnitude sufficient to carry out laparoscopic operation using the trocar 50 in this manner.

Now, the applicator 10 is described.

The applicator 10 includes a nozzle 12 and a sheath 14 into which the nozzle 12 is inserted. The nozzle 12 includes a spray head 20, a nozzle main body 22 in the form of a pipe, and a nozzle head 24. The nozzle head 24 is curved in a state in which no external force is applied thereto.

The spray head 20 has a substantially pentagonal shape as viewed in plan and has the nozzle main body 22 connected to an apex angle portion thereof. On the nozzle main body 22, the nozzle head 24 is provided at an end portion (hereinafter referred to also as distal end portion) at the opposite side to the side at which the spray head 20 is provided. The nozzle main body 22 and the nozzle head 24 are fitted for relative movement along a longitudinal axial direction thereof in the sheath 14.

Note that, for example, the length of the nozzle main body 22 is 30 cm; the outer diameter of the nozzle main body 22 is 3.7 mm; and the inner diameter of the sheath 14 is 4.5 mm. Therefore, a gap 14d of 0.4 mm is defined between an inner face 14a of the sheath 14 and the nozzle main body 22. The gap 14d functions as an exhaust path along which the gas G_{L} in the abdominal cavity 72 is to be exhausted to the outside of the body when the pressure in the abdominal cavity 72 rises. The gas G_{L} in the abdominal cavity 72 passes the gap 14d from a distal end portion 14b of the sheath 14 and is exhausted to the outside of the body past a proximal end portion 14c of the sheath 14. In this case, the distal end portion 14b of the sheath 14 functions as an intake port for gas, and the proximal end portion 14c of the sheath 14 functions as a gas leak exit.

The sheath 14 is configured from an elongated pipe body open at the opposite ends thereof, and part of the nozzle head 24 and the nozzle main body 22 are fitted in the inside of the sheath 14. In the present embodiment, the sheath 14 extends from a location on the distal end side with respect to the curved portion of the nozzle head 24 to a location in the proximity of the connection location of the nozzle main body 22 to the spray head 20. Further, the sheath 14 is movable along the longitudinal axial direction of the nozzle main body 22 relative to the nozzle main body 22 and the nozzle head 24.

Further, the sheath 14 has a function as a shape regulation member for regulating the shape of the curved portion of the nozzle main body 22 as hereinafter described.

The sheath 14 has a plurality of side holes formed on a circumference thereof at a plurality of formation positions in the longitudinal axial positions spaced by equal distances from each other along the longitudinal direction thereof. More particularly, two side holes 15a are formed at positions at the distal end portion 14b side of the sheath 14 along the longitudinal direction, and two side holes 15b are formed at positions at the proximal end portion 14c side of the sheath 14.

Here, the side holes 15a and 15b are formed such that the distance between the distal end portion 14b of the sheath 14 and the formation position of the side holes 15a, the distance between the formation positions of the side holes 15a and the side holes 15b and the distance between the formation position of the side holes 15b and the proximal end portion 14c are equal to each other along the longitudinal axial direction of the sheath 14.

At the formation positions in the longitudinal axial direction, the two side holes 15a and the two side walls 15b are formed at an equal distance in a circumferential direction of the sheath 14. In other words, the two side holes 15a and the two side holes 15b are formed in an opposing relationship on a circumference at the formation positions in the longitudinal axial direction.

The side holes 15a and 15b extend through the sheath 14 and are communicated with the gap 14d, and function as inlets through which the gas G_{L} in the abdominal cavity 72 flows into the gap 14d, namely, as intake ports through which the gas G_{L} in the abdominal cavity 72 is taken in.

If, for example, the total length of the sheath 14 is 30 cm as depicted in FIG. 2, then the side holes 15a are formed at an interval of 10 cm along the longitudinal axial direction of the sheath 14 and the side holes 15b are formed at a position of 20 cm from the distal end portion 14b. The interval at which the side holes are formed is not limited to 10 cm but may be 5 cm.

The gas leak amount varies depending upon the distance between the side holes and the proximal end portion 14c which functions as a gas leak exit. As the distance to the proximal end portion 14c decreases, the gas leak amount increases. Therefore, the gas leak amount is greater from the side holes 15b than from the side holes 15a.

By forming the plurality of side holes 15a and the plurality of side holes 15b at the plurality of positions of the sheath 14 along the longitudinal axial direction, in addition to a route along which the gas G_{L} in the abdominal cavity 72 passes from the distal end portion 14b of the sheath 14 through the gap 14d and is exhausted to the outside of the body through the proximal end portion 14c (gas leak exit) of the sheath 14, another route is formed if the side holes exist in the abdominal cavity 72. In particular, along the latter route, the gas G_{L} in the abdominal cavity 72 passes through the side holes and are exhausted to the outside of the body past the gap 14d and the proximal end portion 14c.

On the other hand, when the side holes are positioned in the trocar 50, a further route is formed. In particular, along the further route, the gas G_{L} in the abdominal cavity 72 enters the main body 52 of the trocar 50 through a distal end portion 52a, passes the gap 53 between the main body 52 and the sheath 14 and further passes the side holes 15b and the gap 14d of the sheath 14 and is then exhausted to the outside of the body from the proximal end portion 14c.

By forming the above-described side holes in this manner, a plurality of exhaust routes of the gas G_{L} in the abdominal cavity 72 to the outside of the body are obtained in addition to the route from the distal end portion 14b described hereinabove. The applicator 10 of the present embodiment has such a gas leak function for exhausting the gas G_{L} in the abdominal cavity 72 to the outside of the body as described above.

The two side holes 15a formed at the distal end portion 14b side of the sheath 14 are formed with a size with which the total area thereof is 6.28 mm² for example, in order to assure a leak flow amount of 2 to 4 L/min when the intraperitoneal pressure is 8 to 12 mmHg. Also the two side holes 15b formed at the proximal end portion 14c side of the sheath 14 are formed with a size with which the total area thereof is 6.28 mm².

If the total area is greater, then the leak flow amount exceeds 4 L/min. In the trocar 50, in order to maintain the intraperitoneal pressure of 8 to 12 mmHz for the leak amount of the gas G_{L} in the abdominal cavity 72, the sterile gas Gv is supplied from the gas supplying unit 60 under the control of the control unit. However, if the leak flow amount exceeds 4 L/min, then the supply amount becomes greater.

On the other hand, if the total area is smaller, then the leak flow amount becomes lower than 2 L/min, and there is the possibility that, at the time of treatment in which the applicator 10 is used, the intraperitoneal pressure may rise exceeding 8 to 12 mmHg.

In the present embodiment, preferably the size of the side holes 15a and 15b is equal to or smaller than 3 mm in diameter where the leak flow amount is taken into consideration. Particularly preferably, the size is 2 mm in diameter.

Further, although the number of side holes at the same formation position is two in the present embodiment, the number is not limited to two but may be three or more. Note that, if the number of side holes provided at the same formation position is one, then the nozzle main body 22 may be one-sided and brought into contact with the inner face 14a of the sheath 14, and thereupon, the side hole may possibly be closed up. In this case, the closed up side hole no more functions as the exhausting route of the gas G_{L} in the abdominal cavity 72. Therefore, the number of side walls at the same formation position is 2 or more.

Note that the formation positions of the side holes in the longitudinal axial direction of the sheath 14 and the number of side holes in a circumferential direction of the sheath 14 at each of the same formation positions and so forth are not limited particularly but can be determined suitably if the intraperitoneal pressure can be held at 8 to 12 mmHg and the leak flow amount can be made 2 to 4 L/min.

The sheath 14 is configured from a material which can regulate the shape of the curved portion of the nozzle head 24 when the curved portion of the nozzle head 24 is covered partly or entirely with the sheath 14. As the constituent material of the sheath 14, for example, polyethylene is used.

On the spray head 20 which configures the nozzle 12, a first connection portion 30a to be connected to a first syringe 34a and a second connection portion 30b to be connected to the second syringe 34b are provided at the opposite side to the side at which the nozzle main body 22 is provided.

A first inner pipe 32a is connected to the first connection portion 30a. The first inner pipe 32a is provided for jetting first liquid supplied thereto from the first syringe 34a from the nozzle head 24. The first inner pipe 32a is fitted in the nozzle main body 22 and further connected to the nozzle head 24.

A second inner pipe 32b is connected to the second connection portion 30b. The second inner pipe 32b is provided for getting second liquid supplied thereto from the second syringe 34b from the nozzle head 24. The second inner pipe 32b is fitted in the nozzle main body 22 and further connected to the nozzle head 24.

The first syringe 34a and the second syringe 34b are connected to a pushing unit 36. The pushing unit 36 is provided for pushing the first syringe 34a and the second syringe 34b. The configuration of the pushing unit 36 is not limited specifically and may be of any of the manual operation type and the automatic operation type only if the pushing unit 36 can push the first syringe 34a and the second syringe 34b.

The first syringe 34a and the second syringe 34b are pushed by the pushing unit 36. Consequently, the first liquid can be supplied into the first inner pipe 32a and the second liquid can be supplied into the second inner pipe 32b readily and with certainty. The pushing operation of the pushing unit 36 can be carried out at an arbitrary timing by an operation of the applicator 10 by an operator.

The first liquid filled in the first syringe 34a and the second liquid filled in the second syringe 34b are different in composition from each other.

The first liquid and the second liquid are selected suitably in accordance with an application, an intended usage, a patient and so forth. For example, where the applicator 10 is used for application of anti-adhesion material, for example, one of the first liquid and the second liquid is liquid containing carboxymethyl dextrin which have been modified with a succinimidyl group while the other is liquid containing sodium carbonate and sodium hydrogen carbonate.

On the other hand, where the applicator 10 is used for application of biological tissue adhesive, one of the first liquid and the second liquid is liquid containing thrombin and the other is liquid containing fibrinogen.

If the first liquid and the second liquid of any of such combinations as described above are mixed, then they gelate. As a result of the gelation, for example, the mixture of the first liquid and the second liquid (hereinafter referred to as "mixed solution") can stay at the applied biological tissue (target region) with certainty. Further, since the mixed solution stays at the target region with certainty, it can exhibit its function as the biological tissue adhesive or the anti-adhesion material with certainty at the applied biological tissue (target region).

Note that the types and the combination of the first liquid and the second liquid are not limited to those described above.

A port 29 is provided on the spray head 20 and communicated with the nozzle main body 22. A gas supplying unit 38 is provided for the port 29 through a tube 37. The port 29 functions as a connection port to a gas supply port of the gas supplying unit 38.

The gas supplying unit 38 includes a gas bomb (not depicted) in which sterile gas G is filled at a high pressure. The sterile gas G is provided for jetting mixed solution Lc hereinafter described, and for example, nitrogen gas or the air is used as the sterile gas G.

The sterile gas G can be supplied at a high flow speed to the nozzle head 24 from the gas supplying unit 38. An on-off valve (not depicted) for controlling the sterile gas G between a supply state and a stop state is installed in the gas supplying unit 38 or the tube 37. When the mixed solution Lc hereinafter described is to be applied, the valve is placed into an on state.

Note that the supply unit is configured from the first syringe 34a and the second syringe 34b as well as the pushing unit 36 and the gas supplying unit 38.

The nozzle main body 22 has a shape of a pipe configured, for example, from stainless steel and is configured from a hollow stainless steel shaft. The nozzle main body 22 has a length of, for example, 30 cm. As described hereinabove, in the inside of the nozzle main body 22, the first inner pipe 32a and the second inner pipe 32b are fitted and the sterile gas G passes.

The nozzle head 24 is provided at a distal end portion of the nozzle main body 22. The nozzle head 24 is hollow and has a nozzle portion 26 provided in the inside thereof. The first inner pipe 32a and the second inner pipe 32b are connected to the nozzle portion 26, and the first liquid supplied through the first inner pipe 32a and the second liquid supplied through the second inner pipe 32b are mixed with each other in the nozzle portion 26.

The nozzle portion 26 is inserted partly in an opening 24a of the nozzle head 24, and, for example, the other portion of the nozzle portion 26 than the portion inserted in the opening 24a is formed from a porous material.

Consequently, by an operation of the pushing unit 36, the first liquid and the second liquid are supplied to the nozzle portion 26, and the mixed solution Lc in the nozzle portion 26 can be injected with certainty from the opening 24a by the sterile gas G flowing into the nozzle portion 26 through the inside of the nozzle main body 22 from the gas supplying unit 38. Note that the mixed solution Lc is mixture of the first liquid, second liquid and sterile gas G.

When the nozzle head 24 is jetting the mixed solution Lc, the sterile gas G passing through the nozzle portion 26 becomes microbubbles in the mixed solution which passes through the nozzle portion 26. By the microbubbles, the mixed solution Lc is agitated in the process of passing through the nozzle portion 26. Consequently, the first liquid and the second liquid are mixed uniformly and with certainty and are injected as the mixed solution Lc from the opening 24a. Especially, when the two liquids are different from each other in viscosity, although uniform mixture solution is less likely to be obtained if the liquids are merely merged, by utilizing the microbubbles, an agitation action of agitating the first liquid and the second liquid to promote mixture of them is manifested. Consequently, the uniform mixed solution Lc is obtained.

The nozzle head 24 has flexibility and is curved such that, for example, the distal end of the nozzle head 24 is directed to an oblique upper side. The axial line g₂ of the nozzle head 24 is inclined by a predetermined angle with respect to the axial line g₁ of the nozzle main body 22.

The inclination angle θ of the axial line g₂ of the nozzle head 24 with respect to the axial line g₁ of the nozzle main body 22 when the nozzle head 24 is in a curved state without being regulated by the sheath 14 hereinafter described preferably is approximately 30 to 90 degrees, and more preferably is approximately 70 to 90 degrees.

The curved portion of the nozzle head 24 is configured, for example, from a soft material, an elastic material or the like. Note that a portion of the nozzle head 24 at the proximal end side with respect to the curved portion may be configured from a hard material or else may be configured from a soft material, an elastic material or the like having flexibility.

Further, the nozzle head 24 may be configured such that the curved portion of the nozzle head 24 and the portion of the nozzle head 24 at the proximal end side with respect to the curved portion are configured from separate members and fixed to each other by adhesion, fusion or the like or may be configured otherwise such that the two portions are formed as a unitary member.

The nozzle head 24 may have a configuration disclosed, for example, in the figures 18 to 26 of U.S. Patent Application Publication No. 2009/0124986. Note that, although the nozzle portion 26 is partly configured from a porous material as described above, the nozzle portion 26 is not limited to this and may be entirely configured from a porous material.

The nozzle main body 22 has, for example, two crushed portions 28a and 28b provided at a proximal end portion 28 thereof at the spray head 20 side as depicted in FIGS. 3(a) and 3(b).

Each of the crushed portions 28a and 28b is formed by pressing the proximal end portion 28 of the nozzle main body 22 from the opposite sides of the nozzle main body 22 by a press to deform the proximal end portion 28 of the nozzle main body 22 in a direction orthogonal to the pressing direction in which the nozzle main body 22 is pressed by the press (the direction orthogonal to the pressing direction is hereinafter referred to as deformation direction) leaving a space, in which the first inner pipe 32a and the second inner pipe 32b can be fitted, in the inside of the nozzle main body 22. Each of the crushed portions 28a and 28b is a flattened portion formed by deforming the nozzle main body 22 in a deformation direction as described above.

The crushed portion 28a and the crushed portion 28b are formed contiguously to each other with the pressing directions with respect to the axial line g₁ of the nozzle main body 22 displaced by a predetermined angle α from each other. The displaced angle α is, for example, 90 degrees. In particular, the deformation directions of the crushed portion 28a and the crushed portion 28b are different from each other, and the angle defined by the deformation angles of the crushed portions 28a and 28b is 90 degrees. Note that the displacement angle α and the angle defined by the deformation directions of the crushed portions 28a and 28b are hereinafter referred to also as installation angle.

The crushed portions 28a and 28b contact with the inner face 14a of the sheath 14 to such a degree that sliding resistance is generated, and preferably the crushed portions 28a and 28b have a size which is greater than the inner diameter of the sheath 14 and with which the crushed portions 28a and 28b can push out the outer face of the sheath 14 to the outer side. In this manner, the crushed portions 28a and 28b contact with the inner face 14a of the sheath 14 contiguously to each other in different deformation directions from each other.

Where the crushed portions 28a and 28b are formed in such a manner as described above, if the sheath 14 and the nozzle main body 22 are moved relative to each other in the longitudinal axial direction of the sheath 14 in a state in which the crushed portions 28a and 28b contact with the inner face 14a of the sheath 14, then the crushed portions 28a and 28b and the inner face 14a of the sheath 14 slidably move. Thereupon, frictional resistance is generated between the inner face 14a of the sheath 14 and the adjacent crushed portions 28a and 28b, and required sliding resistance can be obtained.

If the crushed portions 28a and 28b are provided otherwise in a spaced relationship from each other, then when the sheath 14 is deformed, the sliding resistance is lower similarly as in the alternative case in which a single crushed portion is provided. Therefore, preferably the crushed portions 28a and 28b are provided contiguously each other.

In the present embodiment, since the crushed portion 28a and the crushed portion 28b of the nozzle main body 22 are contiguous and connected to each other in different directions of the sheath 14, the deformation directions or increased diameter directions of the sheath 14 at the contacting portions are different from each other. Therefore, the sliding resistance between the nozzle main body 22 and the sheath 14 can be enhanced. For example, even if the sheath 14 is deformed or increased in diameter by heating upon sterilization in which an autoclave is used or by time dependent variation or the like thereof, particularly if the deformation or diameter increase relates to only one of the two directions, then ones of the crushed portions 28a and 28b contiguous to each other can maintain the state in which they contact with the inner face 14a of the sheath 14. Therefore, a drop of the sliding resistance can be suppressed. Consequently, degradation of the operability of the applicator 10 by deformation of the sheath 14 can be suppressed. Besides, the possibility that deformation or diameter increase in one direction may occur before deformation or diameter increase in two different directions may occur is high, and therefore, the applicator 10 can cope also with the time dependent variation and so forth of the sheath 14 and can achieve a stabilized operability over a long period of time.

Note that, even if the sheath 14 is deformed by heating upon sterilization in which an autoclave is used, by time dependent variation or the like of the applicator, if a drop of the sliding resistance can be suppressed, then the number of crushed portions is not limited specifically.

As regards the size of the crushed portions 28a and 28b, for example, the length in the longitudinal axial direction is 3 to 10 mm, and preferably is 4 to 6 mm. Meanwhile, the width of the crushed portions 28a and 28b in a diametrical direction is, for example, greater by 0.1 to 0.9 mm than the inner diameter of the sheath 14, and preferably is greater by 0.2 to 0.6 mm.

Although the effect of the crushed portions 28a and 28b can be exhibited if the number of the crushed portions 28a and 28b is equal to or greater than two, preferably the number is two. If the number of crushed portions 28a and 28b is excessively great, then there is the possibility that the bending strength of the nozzle may drop.

The installation angle of the crushed portions 28a and 28b preferably is 90 ± 30 degrees (60 to 120 degrees), and more preferably is 90 ± 20 degrees (70 to 110 degrees).

Meanwhile, the contiguous interval between the crushed portions 28a and 28b preferably is 2 to 20 mm, and more preferably is 3 to 10 mm. If the interval between the crushed portions 28a and 28b is smaller, then it is difficult to work the crushed portions 28a and 28b. On the other hand, if the interval between the crushed portions 28a and 28b is excessively great, then a drop of the sliding resistance by the time dependent variation cannot be suppressed.

Meanwhile, if the crushed portions 28a and 28b are provided, for example, at an intermediate region of the nozzle main body 22 in the longitudinal axial direction, then the workability is degraded significantly in that the crushed portions 28a and 28b are caught by the opening 56 of the trocar 50 or the like. Therefore, the crushed portions 28a and 28b are provided at the proximal end portion 28 of the nozzle main body 22.

Further, since the crushed portions 28a and 28b have high sliding resistance and the sheath 14 can be moved and stopped and then kept stopped at a predetermined stopping position, the crushed portions 28a and 28b function as positioning means for carrying out positioning of the sheath 14 in the longitudinal axial direction of the nozzle 12 at the stopping position. Consequently, the mixed solution Lc can be jetted in a state in which the nozzle head 24 is kept at the predetermined inclination angle θ.

Further, the applicator 10 is used in a state in which it is inserted in the trocar 50 as described hereinabove. If, in this state, the applicator 10 is pushed in a direction toward the distal end thereof, then outer peripheral portions of the sheath 14 at which the crushed portions 28a and 28b are positioned are abutted with edge portions of the opening 56 of the main body 52. Therefore, a limit to the movement of the sheath 14 in the direction toward the distal end with respect to the trocar 50 can be regulated. Consequently, the sheath 14 of the applicator 10 can be prevented from inadvertently entering the trocar 50, and the crushed portions 28a and 28b function also as regulation means for regulating the limit to the movement of the sheath 14 in the direction toward the distal end with respect to the trocar 50.

In the present embodiment, the sliding resistance of the sheath 14 by the crushed portion 28a and the crushed portion 28b of the nozzle main body 22 preferably is 3.0 to 11.0 N. The crushed portions are formed with the size, number and installation angle thereof determined such that such sliding resistance as just mentioned can be achieved. Note that the sliding resistance of the sheath 14 was measured in the following manner.

The measuring method of the sliding resistance of the sheath is described with reference to FIGS. 4(a) and 4(b). Note that, in FIGS. 4(a) and 4(b), like components to those of the applicator 10 depicted in FIG. 1 are denoted by like reference symbols, and detailed description of them is omitted herein.

First, the measuring method of the sliding resistance of the sheath when the nozzle main body 22 is pulled is described.

As depicted in FIG. 4(a), a fixing jig 80 was installed on an autograph (not depicted), and a flaring unit 82 was used to fix the applicator 10 to the fixing jig 80 with the nozzle head 24 positioned on the lower side. Then, the spray head 20 was grasped by the chuck of the autograph on the load cell side.

Then, the spray head 20 was pulled in accordance with tensile test conditions given below, and maximum force which was generated till a point of time immediately before the nozzle head 24 was brought into contact with the sheath 14 was measured. Then, the maximum force was determined as the sliding resistance value of the sheath 14 when the nozzle main body 22 was pulled.

As the tensile test conditions, the chuck distance D (refer to FIG. 4(a)) was set to 31.5 ± 0.5 mm; the tensile speed was set to 100 mm/min; and the stroke distance was set to 17.0 mm.

Note that the chuck distance D is a chuck distance from the plane of the fixing jig 80 to a protrusion denoted by reference numeral 84. The protrusion 84 corresponds to the port 29 in FIG. 1 and is a connection portion for the gas supply port.

Now, the measuring method of the sliding resistance of the sheath when the nozzle main body 22 is pushed is described.

As depicted in FIG. 4(b), the fixing jig 80 was placed on the autograph (not depicted), and the flaring unit 82 was used to fix the applicator 10 to the fixing jig 80 with the nozzle head 24 positioned on the lower side. Then, the spray head 20 was grasped by the chuck of the autograph on the load cell side.

Then, the spray head 20 was pushed in in accordance with pushing-in conditions given below, and maximum force which was generated till a point of time immediately before the spray head 20 was brought into contact with the sheath 14 was measured. Then, the maximum force was determined as the sliding resistance value of the sheath 14 when the nozzle main body 22 is pushed.

As the pushing-in conditions, the chuck distance D (refer to FIG. 4(b)) was set to 31.5 ± 0.5 mm, and the pushing-in speed was set to 100 mm/min.

In the applicator 10, when the sheath 14 is moved relative to the nozzle main body 22 along the longitudinal axial direction of the nozzle main body 22, the curved portion of the nozzle head 24 is inserted into the sheath 14. By adjusting the projection length of the curved portion of the nozzle head 24 from the distal end of the sheath 14, the shape of the curved portion can be changed. Consequently, the inclination angle θ of the axial line g₂ of the nozzle head 24 with respect to the axial line g₁ of the nozzle main body 22, namely, the direction of the nozzle head 24, can be adjusted. In particular, for example, the sheath 14 is movable between a first position (inclination angle θ = 0 degrees) in which the curved portion of the nozzle head 24 is regulated into a linear shape by the sheath 14 and the direction of the axial line g₂ of the nozzle head 24 and the direction of the axial line g₁ of the nozzle main body 22 coincide with each other and a second position (the inclination angle θ is the maximum inclination angle) in which the curved portion is in the curved state without being regulated by the sheath 14 and the axial line g₁ of the nozzle main body 22 is inclined with respect to the axial line g₂ of the nozzle head 24. Therefore, by moving the relative position of the sheath 14 and the nozzle main body 22 to a predetermined position between the first position and the second position, the inclination angle θ of the nozzle head 24 can be adjusted freely within the range from 0 degrees to the maximum inclination angle.

In the present embodiment, the crushed portions 28a and 28b exert high sliding resistance with respect to the inner face 14a of the sheath 14 and can stop the sheath 14 at a predetermined stopping position as described above. Besides, the crushed portions 28a and 28b have withstanding property against heating upon sterilization and time dependent variation as described above, and the high sliding resistance with the inner face 14a of the sheath 14 can be maintained over a long period of time. Therefore, the curved portion of the nozzle head 24 can be regulated accurately by the sheath 14, and the mixed solution Lc can be applied accurately keeping the inclination angle θ of the nozzle head 24 at the predetermined angle. Further, even if the applicator 10 is used repetitively, the mixed solution Lc can be applied in such a manner that the inclination angle θ of the nozzle head 24 is kept at the predetermined angle stably and accurately over a long period of time.

In this manner, while the sheath 14 is moved to suitably adjust the inclination angle θ to change the inclination angle θ of the nozzle head 24, the mixed solution Lc can be applied in such a manner as described above from the opening 24a of the nozzle head 24 toward a plurality of locations in the abdominal cavity 72, for example, toward internal organs and the abdominal wall 70 over a wide range readily, with certainty and stably over a long period of time.

Note that, in the applicator 10, by suitably setting the degree of the curve (inclination angle θ) of the curved portion thereof in a natural state in which no external force is applied thereto, for example, if the applicator 10 is formed in a "U" shape, then the mixed solution Lc can be applied also to the abdominal wall 70.

Here, FIGS. 5(a) and 5(b) are schematic views illustrating usage patterns of the applicator of the embodiment of the present invention, and FIG. 5(c) is a schematic view of a usage pattern of a conventional applicator. In FIGS. 5(a) and 5(b), like elements to those of the applicator 10 depicted in FIG. 1 are denoted by like reference symbols, and detailed description of them is omitted herein.

As depicted in FIG. 5(a), when the applicator 10 is inserted into the abdominal cavity 72 such that the side holes 15b at the upper side enter the abdominal cavity 72, totaling three exhaust routes for the gas G_{L} in the abdominal cavity 72 are available including a first route which passes the distal end portion 14b of the sheath 14, a second route which passes the side holes 15a, and a third route which passes the side holes 15b. Therefore, if the distal end portion 14b of the sheath 14 is closed up with liquid of ascitic fluid, cleaning solution or the like existing in the abdominal cavity 72, then even if the pressure in the abdominal cavity 72 becomes high as a result of injection of the mixed solution Lc for which the sterile gas G of the applicator 10 is used, the gas G_{L} in the abdominal cavity 72 can be exhausted to the outside of the body through the second route and the third route described above.

On the other hand, when applicator 10 is inserted into the abdominal cavity 72 such that the insertion length of the applicator 10 in the abdominal cavity 72 is short and the side holes 15b at the upper side exist outside the abdominal wall 70 as depicted in FIG. 5(b), totaling two exhaust routes for the gas G_{L} in the abdominal cavity 72 are available including the first route and the second route described above. Therefore, if the distal end portion 14b of the sheath 14 is closed up with liquid of ascitic fluid, cleaning solution or the like existing in the abdominal cavity 72, then even if the pressure in the abdominal cavity 72 becomes high as a result of injection of the mixed solution Lc for which the sterile gas G of the applicator 10 is used, the gas G_{L} in the abdominal cavity 72 can be exhausted to the outside of the body through the second route described above.

In the conventional applicator 100 having no side hole as depicted in FIG. 5(c), only the first route is available as the exhaust route for the gas G_{L} in the abdominal cavity 72. Therefore, if the distal end portion 14b of the sheath 14 is closed up with liquid of ascitic fluid, cleaning solution or the like existing in the abdominal cavity 72, then even if the pressure in the abdominal cavity 72 becomes high as a result of injection of the mixed solution Lc for which the sterile gas G of the applicator 10 is used, the gas G_{L} in the abdominal cavity 72 cannot be exhausted to the outside of the body.

As described above, with the applicator 10 of the present embodiment, even if the depth of the insertion of the applicator 10 varies, the gas leak function is maintained without being influenced by the variation of the insertion length. Therefore, even if the pressure in the abdominal cavity 72 becomes high as a result of injection of the mixed solution Lc for which the sterile gas G of the applicator 10 is used, the gas G_{L} in the abdominal cavity 72 can be exhausted to the outside of the body and the pressure rise in the abdominal cavity 72 can be suppressed. Furthermore, since the applicator 10 includes the plurality of exhaust routes, even if one of the exhaust routes is closed up, the gas leak function is maintained similarly as described above. Consequently, the pressure rise in the abdominal cavity 72 by use of the applicator 10 can be suppressed.

The present invention is configured basically in such a manner as described above. While the applicator of the present invention has been described in detail above, the present invention is not limited to the embodiment described above and it is a matter of course that various improvements or alterations may be made without departing from the subject matter of the present invention as defined in the appended claims.

### Description of Reference Symbols

- 10: Applicator
- 12: Nozzle
- 14: Sheath
- 14d,: 53 Gap
- 15a,: 15b Side hole
- 20: Spray head
- 22: Nozzle main body
- 24: Nozzle head
- 26: Nozzle portion
- 28: End portion
- 28a,: 28b Crushed portion
- 30b: Second connection portion
- 30a: First connection portion
- 34a: First syringe
- 34b: Second syringe
- 36: Pushing unit
- 38,: 60 Gas supplying unit
- 50: Trocar
- 72: Abdominal cavity
- 80: Fixing jig
- Lc: Mixed solution

## Claims

1. An applicator (10), comprising:
a nozzle (12) including an elongated nozzle main body (22) to which gas and a plurality of kinds of liquids can be supplied and a nozzle head (24) provided at a distal end side of the nozzle main body (22) and configured to jet mixed solution of the gas and the plurality of kinds of liquids supplied to the nozzle main body (22); and
a sheath (14) in which the nozzle main body (22) is fitted for relative movement along a longitudinal axial direction of the nozzle main body (22);
the applicator (10) being configured to be inserted into a living body to apply the mixed solution to a region in the living body;
a gap (14d, 53) being provided between the nozzle main body (22) and the sheath (14) so as to function as an exhaust path for exhausting gas in the living body to the outside of the body when the pressure in the living body rises;
**characterized in that** the sheath (14) has a plurality of side holes (15a, 15b) formed on a circumference thereof at a plurality of positions spaced by an equal interval from each other along a longitudinal axial direction of the sheath (14) and from a distal end portion (14b) of the sheath (14) and from a proximal end portion (14c) of the sheath, each of the side holes (15a, 15b) communicating with the gap (14d, 53).

2. The applicator (10) according to claim 1, wherein the plurality of side holes (15a, 15b) are formed at an equal interval along a circumferential direction of the sheath (14) at each of the plurality of positions along the longitudinal axial direction of the sheath (14).

3. The applicator (10) according to claim 1 or 2,
wherein the number of the plurality of side holes (15a, 15b) formed along the circumferential direction of the sheath (14) at each of the plurality of positions along the longitudinal direction of the sheath (14) is two or three.

## Patentansprüche

1. Applikator (10); umfassend:
eine Düse (12), die einen langgestreckten Düsenhauptkörper (22) aufweist, dem Gas und eine Mehrzahl von Arten von Flüssigkeiten zugeführt werden können, und einen Düsenkopf (24), der an einer distalen Endseite des Düsenhauptkörpers (22) vorgesehen und zum Ausstoßen einer gemischten Lösung des Gases und der Mehrzahl von Arten von Flüssigkeiten konfiguriert ist, die dem Düsenhauptkörper (22) zugeführt werden; und
eine Ummantelung (14), in welcher der Düsenhauptkörper (22) für eine relative Bewegung entlang einer Längsachsenrichtung des Düsenhauptkörpers (22) eingepasst ist;
wobei der Applikator (10) konfiguriert ist, in einen lebenden Körper eingeführt zu werden, um die gemischte Lösung auf einen Bereich in dem lebenden Körper anzuwenden;
einen Spalt (14d, 53), der zwischen dem Düsenhauptkörper (22) und der Ummantelung (14) vorgesehen ist, um so als ein Abgaspfad zum Abführen von Gas in dem lebenden Körper zur Außenseite des Körpers zu wirken, wenn der Druck in dem lebenden Körper ansteigt;
**dadurch gekennzeichnet, dass** die Ummantelung (14) eine Mehrzahl von Seitenlöchern (15a, 15b) aufweist, die an einem Umfang davon an einer Mehrzahl von Positionen ausgebildet sind, welche durch einen gleichen Abstand entlang einer Längsachsenrichtung der Ummantelung (14) voneinander beabstandet sind, und
von einem distalen Endabschnitt (14b) der Ummantelung (14) und von einem proximalen Endabschnitt (14c) der Ummantelung, wobei jedes der Seitenlöcher (15a, 15b) mit dem Spalt (14d, 53) in Verbindung steht.

2. Applikator (10) nach Anspruch 1, wobei die Mehrzahl der Seitenlöcher (15a, 15b) in einem gleichen Abstand entlang einer Umfangsrichtung der Ummantelung (14) an jeder der Mehrzahl von Positionen entlang der Längsachsenrichtung der Ummantelung (14) ausgebildet sind.

3. Applikator (10) nach Anspruch 1 oder 2,
wobei die Anzahl der Mehrzahl von Seitenlöchern (15a, 15b), die entlang der Umfangsrichtung der Ummantelung (14) an jeder der Mehrzahl der Positionen entlang der Längsrichtung der Ummantelung (14) ausgebildet sind, zwei oder drei beträgt.

## Revendications

1. Applicateur (10), comprenant :
une buse (12) comportant un corps principal de buse allongé (22) auquel un gaz et une pluralité de types de liquides peuvent être fournis et une tête de buse (24) prévue au niveau d'un côté d'extrémité distale du corps principal de buse (22) et conçue pour projeter une solution mélangée du gaz et de la pluralité de types de liquides fournis au corps principal de buse (22) ; et
une gaine (14) dans laquelle le corps principal de buse (22) est monté en vue d'un mouvement relatif le long d'une direction axiale longitudinale du corps principal de buse (22) ;
l'applicateur (10) étant conçu pour être inséré dans un corps vivant afin d'appliquer la solution mélangée sur une région du corps vivant ;
un espace (14d, 53) étant prévu entre le corps principal de buse (22) et la gaine (14) de manière à servir de passage d'évacuation pour décharger le gaz présent dans le corps vivant à l'extérieur du corps lorsque la pression dans le corps vivant augmente ;
**caractérisé en ce que** la gaine (14) comprend une pluralité d'orifices latéraux (15a, 15b) formés sur sa circonférence en une pluralité de positions espacées à un intervalle régulier les unes des autres le long d'une direction axiale longitudinale de la gaine (14) et d'une partie d'extrémité distale (14b) de la gaine (14) et d'une partie d'extrémité proximale (14c) de la gaine (14), chacun des orifices latéraux (15a, 15b) communiquant avec l'espace (14d, 53).

2. Applicateur (10) selon la revendication 1, dans lequel la pluralité d'orifices latéraux (15a, 15b) sont formés à un intervalle régulier le long d'une direction circonférentielle de la gaine (14) au niveau de chacune de la pluralité de positions le long de la direction axiale longitudinale de la gaine (14).

3. Applicateur (10) selon la revendication 1 ou 2, dans lequel le nombre de la pluralité d'orifices latéraux (15a, 15b) formés le long de la direction circonférentielle de la gaine (14) au niveau de chacune de la pluralité de positions le long de la direction longitudinale de la gaine (14) est de deux ou trois.
